# EUROPEAN PATENT SPECIFICATION

(11) **EP 1 790 297 B1**
(45) Date of publication and mention of the grant of the patent: **07.10.2015**
(21) Application number: 06256090.9
(22) Date of filing: 28.11.2006
(51) Int. Cl.: A61B 17/11, A61B 17/00

(54) **Anastomosis device**
Vorrichtung für Anastomose
Dispositif d'anastomose

(30) Priority: 29.11.2005 US 164574
(43) Date of publication of application: 30.05.2007
(73) Proprietor: ETHICON ENDO-SURGERY, INC., Cincinnati, Ohio 45242 (US)
(72) Inventor: Ortiz, Mark S., Milford Ohio 45150 (US); Simms, Robert Jason, Liberty Township Ohio 45044 (US); Ditter, Tom Allen, Fremont California 94538 (US); Plescia, David Nicolas, Cincinnati Ohio 94303 (US); Eke, Soane H., Palo Alto California 94303 (US)
(74) Representative: Tunstall, Christopher Stephen

(56) References cited:
- EP-A1- 0 326 757
- WO-A-01/30230
- WO-A-01/95783
- US-A1- 2003 032 967
- US-A1- 2005 143 758
- US-B1- 6 494 889

## Description

### FIELD OF THE INVENTION

The invention relates broadly to an anastomosis device, and more particularly to an anastomosis device formed from a woven tube of shape-memory wire where, when deployed, the anastomosis device can hold tissues of an anastomotic site in apposition.

### BACKGROUND OF THE INVENTION

During many surgical procedures, the surgeon will have to close or ligate various blood vessels and other ducts before severing them in order to prevent excessive bleeding, and reduce the risk of other complications to the patient. One ligation technique is to tie a suture about the vessel to close the vessel. Alternatively, a surgeon can place a clip having a pair of legs connected at their proximal ends about the vessel, and urge or squeeze the legs together to close the vessel.

One drawback associated with some current clips used for ligating vessels is that the legs of the clip may tend to separate to some extent following release from a clip applier. This phenomenon is called duck-billing. Duck-billing can result in insufficient ligation of a vessel, thus leading to excessive blood loss and/or unnecessary damage to the vessel. Further, some known ligation clips are often difficult to preload into a clip applier because of resistance between the tissue disposed between the jaws and the gripping features on the clip legs.

U.S. Patent Application Publication No. 2003/0120292 discloses an anastomosis devices having a woven tube of wire constructed from shape memory alloy and the ends of the tube thermally deforming and everting to form petals in a manner which holds the luminal interface of the anastomotic site into apposition.

Accordingly, there remains a need for an improved surgical instrument and method, and in particular for surgical clips used for ligating blood vessels, other ducts, and the like.

### SUMMARY OF THE INVENTION

Embodiments of the present invention generally provide anastomosis devices for securing layers of tissue, such as the walls of a small intestine and an upper stomach pouch, in apposition. An anastomosis device according to the present invention is defined in appended claims 1 and 3.

An anastomosis device includes a body that is formed from a woven shape-memory wire having a central lumen. The body is configurable between an expanded position, in which the body can assume a generally tubular configuration having a wire mesh wall adapted for insertion into a lumen of an anastomotic site, and a rest position, in which the body can assume an annular configuration having a plurality of petals with petal tips that define an outer periphery. The rest position is effective to hold opposed tissues of the anastomotic site in apposition and can apply a pressure to the opposed tissues such that the pressure decreases from an outer periphery to an inner periphery of the device. The plurality of petals are configured to apply the pressure to the opposed tissues to cause necrosis of the opposed tissues in a contact region, such as a region about the outer periphery of the device. In one embodiment, in the rest position, the tips of the petals are adapted to contact the opposed tissues along a distance greater than approximately 5% of the circumference of the outer periphery of the device.

In accordance with the present invention, the plurality of petals include a superior set of adjacent petals and an inferior set of adjacent petals where the device is adapted to receive the opposed tissues between the inferior and superior sets of petals. In a rest position, each petal of the superior set and inferior set of adjacent petals is formed by adjacent arms connected by a tip that extends along a portion of the outer periphery of the device. Each petal tip has a first radius and each arm is connected to the tip at a bend having a second radius, where the first radius is greater than the second radius. The tips of the superior set of adjacent petals may have midpoints that can be staggered about a circumference of the device relative to midpoints of the tips of the inferior set of adjacent petals or that can be substantially aligned with midpoints of the tips of the inferior set of adjacent petals.

The superior and inferior petals of the device can be configured with a variety of geometries.

In one embodiment not claimed, when the device is configured in the rest-position, the adjacent arms and tip of each petal can be oriented in a substantially planar configuration in a plane that is substantially perpendicular to a central axis extending through the central lumen of the device.

In accordance with the invention, the adjacent arms of each petal are oriented in a plane that is substantially perpendicular to a central axis extending through the central lumen of the device where at least a portion of the adjacent arms of the superior set of petals include an arc portion having an inferior facing opening, and at least a portion of the adjacent arms of the inferior set of petals include an arc portion having a superior facing opening. The adjacent arms of the superior set of petals can include a bend portion disposed between the arc portion and the tip that can orient the tip in the plane that is substantially perpendicular to a central axis extending through the central lumen of the device and the adjacent arms of the inferior set of petals can include a bend portion disposed between the arc portion and the tip. The bend portion of the adjacent arms can orient the tip in the plane that is substantially perpendicular to a central axis extending through the central lumen of the device.

Also in accordance with the invention, the tip of each petal has a peak portion that defines an inferior facing opening and a trough portion that defines a superior facing opening.

In another embodiment not claimed, the anastomosis device can include a first wire circumferentially coupled to the petal tips of the superior set of adjacent petals and a second wire circumferentially coupled to the petal tips of the inferior set of adjacent petals. When the device is configured in the rest position, the first wire and the second wire can contact the opposed tissues along a distance greater than approximately 90% of the circumference of the outer periphery of the device.

### BRIEF DESCRIPTION OF THE DRAWINGS

The invention will be more fully understood from the following detailed description taken in conjunction with the accompanying drawings, in which:

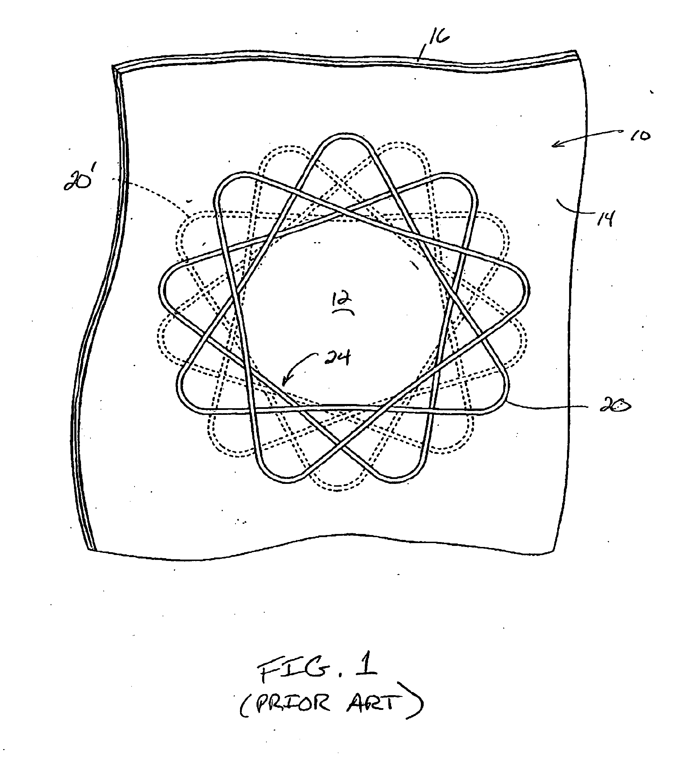

FIG. 1 is a top view of a prior art anastomosis device shown in a deployed state;
FIG. 2 is a side view of one embodiment of a tissue-necrosing type anastomosis device in an expanded elongate tubular configuration;
FIG 3A. is a top view of one embodiment of a tissue-necrosing type anastomosis device in a deployed state having staggered petals with non-overlapping tips;
FIG. 3B is a side view of the anastomosis device of FIG. 3A;
FIG. 3C is a perspective view of the anastomosis device of FIG. 3A having a tissue material disposed between a portion of the opposed petals;
FIG. 4A is a top view of one embodiment of a tissue-necrosing type anastomosis device in a deployed state having staggered overlapping petals;
FIG. 4B is a side view of the tissue-necrosing type anastomosis device of FIG. 4A;
FIG. 4C is a perspective view of the embodiment of the tissue-necrosing type anastomosis device of FIG. 4A having a tissue material disposed between a portion of the opposed petals;
FIG. 4D is a side view of another embodiment of the tissue-necrosing type anastomosis device of FIG. 4A;
FIG. 5A is a top view of one embodiment of a tissue-necrosing type anastomosis device in a deployed state having staggered overlapping petals;
FIG. 5B is a side view of the anastomosis device of FIG. 5A;
FIG. 5C is a perspective view of the anastomosis device of FIG. 5A having a tissue material disposed between a portion of the opposed petals;
FIG. 6A is one embodiment of a tissue-necrosing type anastomosis device in a deployed state having aligned overlapping petals;
FIG. 6B is a side view of the anastomosis device of FIG. 6A;
FIG. 6C is a perspective view of the anastomosis device of FIG. 6A having a tissue material disposed between a portion of the opposed petals;
FIG. 7 illustrates an embodiment of a tissue-necrosing type anastomosis device in a deployed state having a pressure distribution ring disposed about the tips of the petals;
FIG. 8 illustrates the anastomosis device of FIG. 7 prior to formation of the pressure distribution rings;
FIG. 9A is a top view of one embodiment of an anastomosis device configured to promote growth of tissue about an inner periphery of the device;
FIG. 9B is a perspective view of the anastomosis device of FIG. 9A; and
FIG. 9C is a perspective view of the anastomosis device of FIG. 9A deployed at an anastomosis site.

### DETAILED DESCRIPTION OF THE INVENTION

Certain exemplary embodiments will now be described to provide an overall understanding of the principles, structure, function, manufacture, and use of the devices and methods disclosed herein. One or more examples of these embodiments are illustrated in the accompanying drawings. Those skilled in the art will understand that the devices specifically described herein and illustrated in the accompanying drawings are non-limiting exemplary embodiments and that the scope of the present invention is defined solely by the claims.

The present invention generally provides an anastomosis device that can be used to couple two or more layers of tissue in apposition. In an exemplary embodiment, the device can be formed from one or more woven wires that can be configured to have a generally tubular shape in a first, expanded position for insertion to an anastomosis site and a generally annular, ring-shape in a second, resting or deployed position for securing tissue in apposition at the anastomosis site. The device can be adapted to cause necrosis of the tissue layers around an outer periphery of the device.

The device can be formed from a variety of materials, but in an exemplary embodiment, it is formed from a shape-memory wire woven into a mesh. The shape-memory wire is formed into an annular ring such that, when the anastomosis device is expanded into an elongate tubular form, it will return to its annular, ring-shaped configuration that is necessary during use of the device. Suitable shape-memory materials include, by way of non-limiting example, a shape-memory metal, such as an alloy of titanium and nickel (e.g., nitinol), that changes its shape upon the application of a force, such as a tension, and that returns to its deployed state upon removal of the force. A person skilled in the art will appreciate that the wire forming the anastomosis device can also be formed from other materials as well. For example, the wire can be formed from a spring material or from a compressible wire material. The device can also be formed from superelastic metal materials, such as alloys of titanium and nickel, that have the ability to undergo a relatively large elastic deformation when mechanically loaded. Additionally, one skilled in the art will understand that the wire can have a variety of cross-sectional shapes and thicknesses or diameters. For example, the wire can have a round, square, or hexagonal shape and can have a thickness or diameter in the range of about 0.203 mm to 0.584 mm (0.008 inches to 0.023 inches).

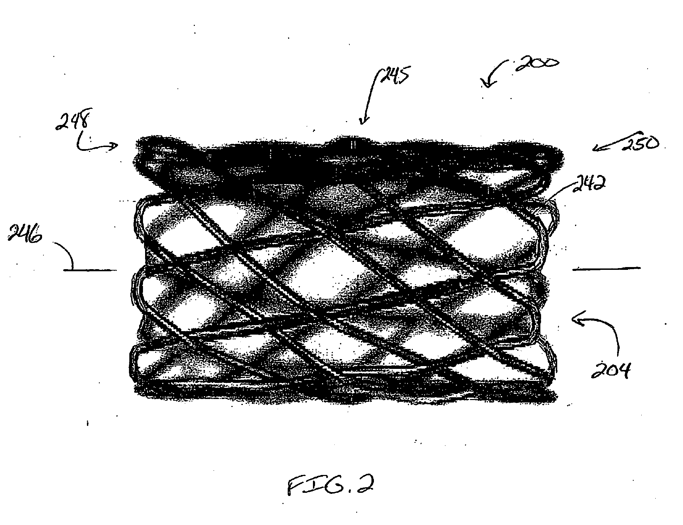

As indicated above, the present invention provides both necrosing and non-necrosing anastomosis devices. FIGS. 2-7 show various exemplary embodiments of anastomosis devices that are adapted to cause necrosis of tissue at an anastomosis site. In general, each device has a ring shaped configuration in a resting or deployed state with an inner and outer periphery. The inner and outer peripheries are configured such that, in the deployed state, pressure is highest at the outer periphery of the deployed device and decreases from the outer periphery to the inner periphery of the device. The relatively high pressure can impede blood flow to the tissue about the outer periphery, thereby causing the tissue inside the outer periphery of the device to become necrotic in this region. As the tissue necroses over time, the device can become separated from the healthy tissue at the anastomosis site and can be passed through the digestive system of the patient.

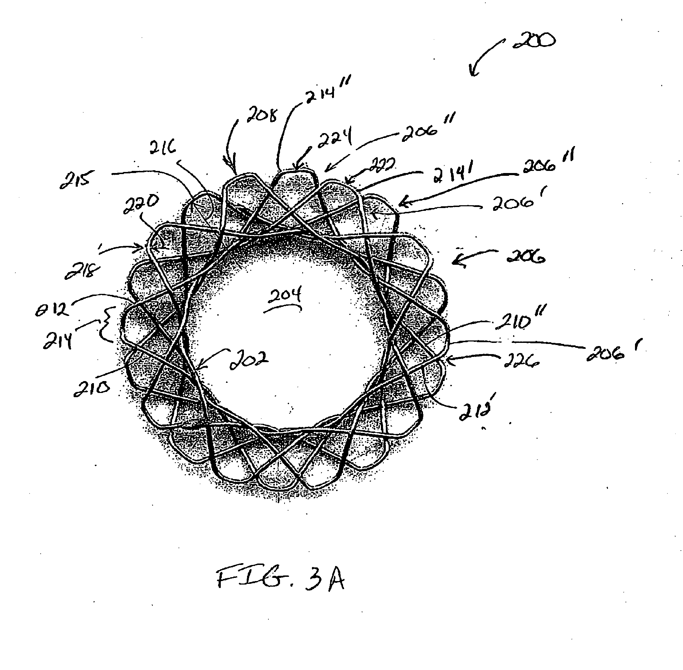

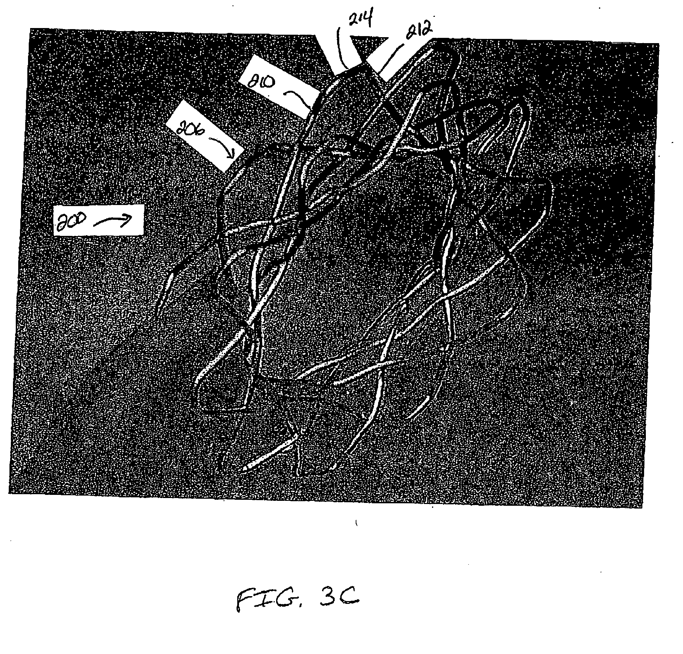

FIG. 2 illustrates one embodiment of an anastomosis device 200 in the expanded elongated tubular configuration, which can be achieved by applying a force, such as a tension, to the device. As shown, the device 200 has a wire mesh wall 242 that includes first 248 and second 250 ends and a midportion 245 disposed between the ends 248, 250. The wire mesh wall 242 also defines a central lumen or opening 204 extending along a central or longitudinal axis 246. The central lumen 204 allows the device 200 to be disposed on the shaft of a delivery device to be delivered to an anastomosis site. As the anastomosis device 200 is deployed from the delivery device, the device 200 will collapse from the elongated state to a resting state to secure opposed tissue layers in apposition. In particular, the wire mesh wall 242 of the tube will contract in the direction of the longitudinal axis 246, the midportion 245 of the device will collapse inward to form an inner periphery of the ring, and ends 248, 250 of the tube will evert to form an outer periphery of the ring. The device 240 will thus have a generally annular configuration in the resting or deployed position, as shown in FIGS. 3A-3C. In particular, the anastomosis device 200 has an annular, ring-shaped configuration with an inner periphery 202 that defines an opening 204 and petals 206 extending from the inner periphery 202 and defining an outer periphery 208. The inner periphery 202 of the device 200 is defined by overlapping of the meshed wire and the outer periphery 208 is defined by the opposed ends of the tube which form opposing superior and inferior petals 206', 206". In the deployed state as shown, the inner periphery 202 can maintain a passageway between two apposed tissue layers at an anastomosis site to allow fluid and/or other substances to be passed between the layers and the superior and inferior petals 206', 206" can engage and apply pressure to tissue captured therebetween. In an exemplary embodiment, the superior and inferior petals 206', 206" can be configured to apply pressure to particular regions of tissue disposed therebetween to facilitate necrosis.

In the embodiment shown in FIGS. 3A-3C, the petals 206 are adapted to apply a pressure to tissue such that the pressure is highest at the outer periphery 208 and decreases from the outer 208 to the inner periphery 202. In particular, each petal 206 can be formed by adjacent arms 210, 212 connected by a tip 214 that extends along a portion of the outer periphery 208 of the device 200. The tip 214 has a bend 215 with a first radius 216, and each arm 210, 212 is connected to the tip 214 at a bend 218 that has a second radius 220. The first radius 216 is greater than the second radius 220. By way of example, the bend 215 can have a radius 216 of approximately 11.18 mm (0.44 inches), while the bends 218, which can form transitions between each arm 210, 212 and the tip 214, have a radius 220 of approximately 1.02 mm (0.04 inches).

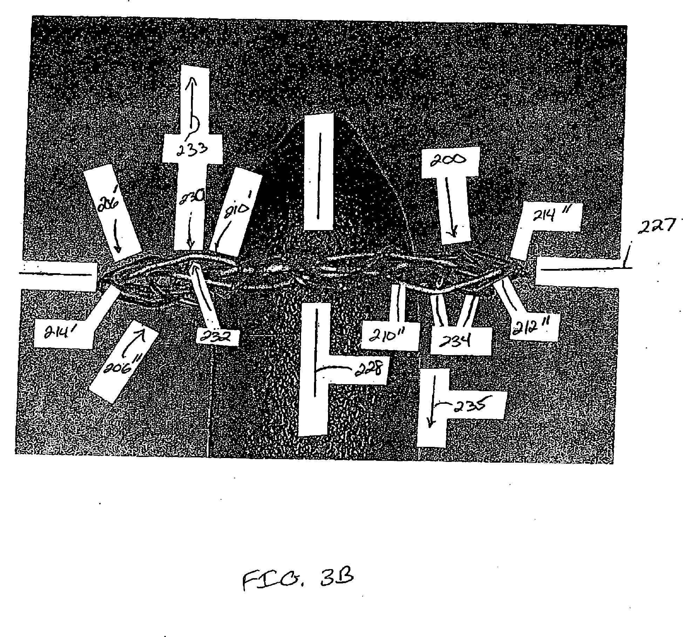

Additionally, as indicated in FIG. 3B, the adjacent arms 210, 212 of each petal 206 can be curved relative to a plane 227 of the device 200 (e.g., in the deployed state) that is substantially substantially perpendicular to a central axis 228 extending through the opening or lumen 204 of the device 200. For example, each of the arms 210', 212' forming each of the superior petals 206' can include an arc portion 230 that extends away from the plane 227 along a first longitudinal direction 233 and that defines an opening 232 that faces (e.g., opposes) the inferior set of petals 206". Additionally, each of the arms 210", 212" forming each of the inferior petals 206" can include an arc portion 234 that extends away from the plane 227 along a second longitudinal direction 235 and that defines an opening 236 that faces (e.g., opposes) the superior set of petals 206'. The arc portions 230, 234 of the opposed petals 206', 206" are configured to orient the respective petal tips 214', 214" relative to the plane 227 of the device 200 such that the petal tips 214', 214" cross the plane 227 of the device 200 (e.g., where the tips 214' of the superior petals 206' extend below the plane 227 and the tips 214" of the inferior petals 206" extend above the plane 227 of the device 200). In use, the curved arc to the arms 210, 212 of the petals 206 can help to focus pressure applied by the petals 206 to the outer periphery 208 of the device 200.

The superior and inferior petals 206', 206" can also be positioned at particular locations relative to one another to effect tissue necrosis at the outer periphery 208 of the device 200. As shown in FIG. 3A, for example, the tips 214 of the superior and inferior petals 206', 206" can be staggered relative to each other about the outer periphery of 208 to distribute pressure to tissue about the circumference or outer periphery 208 of the device 200. In particular, the tips 214' of each of the superior petals 206' have midpoints 222 that are staggered or offset from the midpoints 224 of the adjacent tips 214" of the inferior petals 206". While the midpoints 222, 224 of adjacent, opposed petals 206', 206" can be staggered by any amount, in one embodiment, the midpoints 222, 224 are staggered by an amount in the range of about 15° to 20°, and more preferably by about 18°. As further illustrated in FIG. 3A, the petals 206 are configured such that the tips 214 of opposed, adjacent superior and inferior petals 206', 206" do not cross each other, however the arms 210, 212 forming each of the petals 206 are configured to cross at multiple locations about the circumference of the device 200. For example, an arm 212' of a superior petal 206' crosses an arm 210" of an inferior petal 206" to form a crossing location 226. When the device 200 is deployed at an anastomosis site, the arms 210, 212 of the opposing petals tip 214 is able to contact and apply pressure to the tissue disposed between adjacent locations 226 to limit or prevent blood flow thereto and to cause the tissue to become necrotic.

The number and size of petals 206 can also vary to obtain a desired result. In the embodiment illustrated in FIGS. 3A-3C, the device 200 includes ten superior petals and ten inferior petals. However, other numbers of petals 206 can be used. For example, a device can be formed with fewer superior and inferior petals 206', 206" to increase the stiffness of the device 200 and thus increase the amount of pressure applied by the device 200 on tissue at the outer periphery of the device 200. In another example, a device can be formed with additional superior and inferior petals 206', 206" to decrease the stiffness of the device 200 and thus decrease the amount of pressure applied by the device 200 on tissue at the outer periphery of the device 200.

The anastomosis devices described herein can be deployed using a delivery device of the type known in the art, such as that described in U.S. Patent Application Publication No. 2003/0120292. In use, such as in a side-to-side intestinal anastomosis, the anastomosis device 200 can be expanded into an elongate tubular configuration, such as illustrated in FIG. 2, and disposed about a delivery device. The delivery device can then be inserted within a patient and advanced intraluminally to an anastomosis site. At the anastomosis site, two tissue layers, such as a tissue wall forming an upper stomach pouch and a tissue wall forming a portion of the patient's small intestine, can be brought into apposition. Openings can then be formed within the walls of the tissue such as by a retractable cutting instrument associated with the delivery device. The anastomosis device 200 can then be deployed from the delivery device at the juncture of the apposed openings such that the device 200 collapses from the elongated state to the resting, annular, ring-shaped configuration with the superior petals 206' and the inferior petals 206" disposed on opposite sides of the two tissue layers.

Once the anastomosis device 200 has collapsed from the elongated to the resting state, opposing arms 210, 212 of adjacent petals 206 apply a relatively large pressure to the tissue disposed between the superior and inferior petals 206', 206" at locations 226, thereby limiting or preventing blood from flowing to these tissue regions. Additionally, the tips 214 of each petal 206 can apply a pressure to the tissue disposed at the outer periphery 208. For example, each tip 214 can apply a load to the tissue disposed between adjacent locations 226 at the outer periphery 208 to limit or prevent blood from flowing to the tissue disposed at the outer periphery 208 of the device 200. Over time, as a result of the pressure applied by the petals 206 on the tissue, the tissue can become necrotic at the outer periphery 208 and inbound of the outer periphery 208, thereby allowing the device to become separated from the healthy tissue at the anastomosis site and passed through the digestive system of the patient.

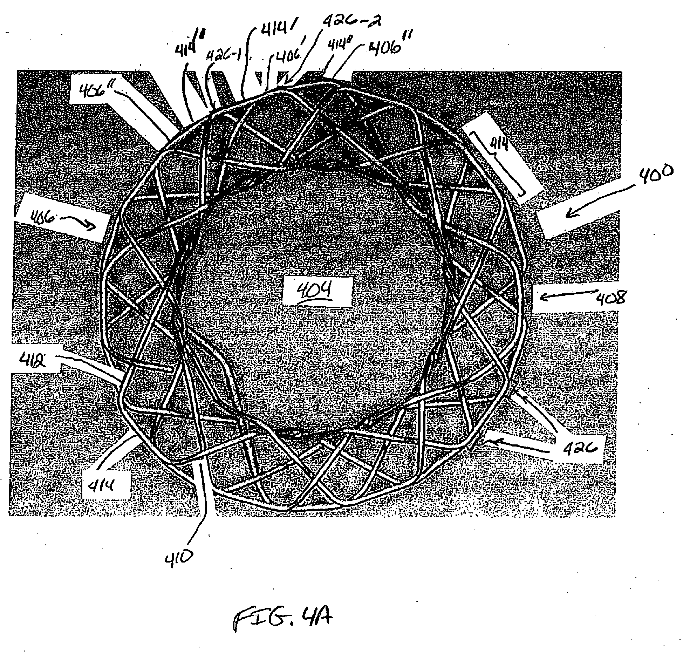

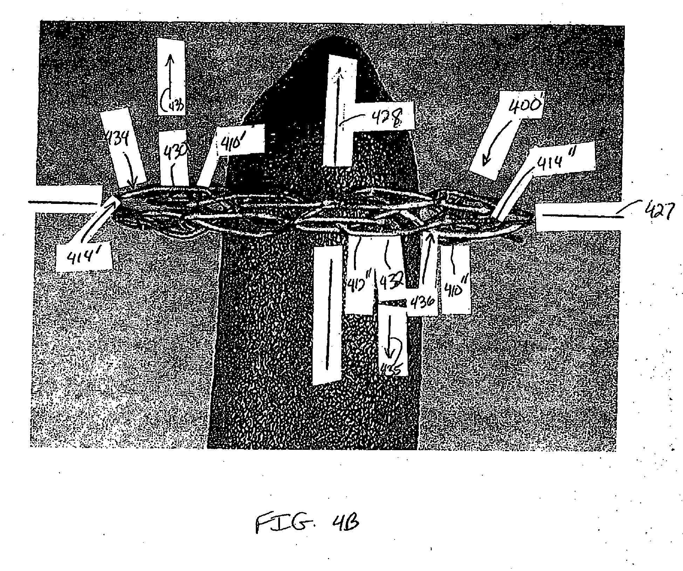

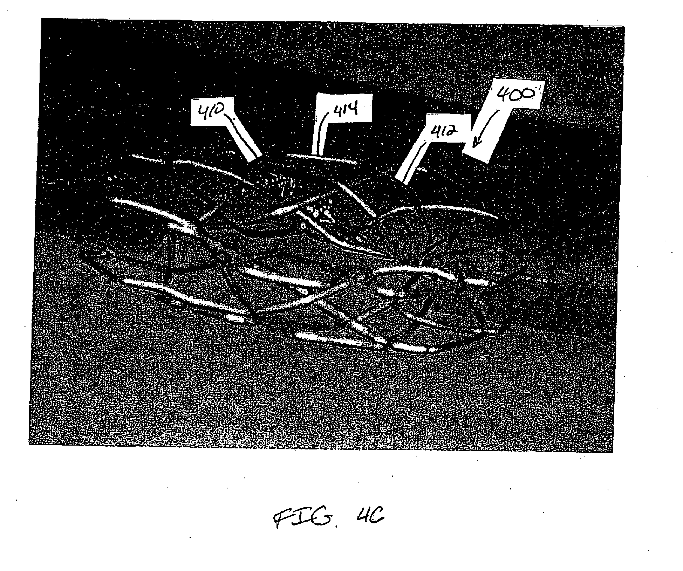

FIGS. 4A-4C illustrate another embodiment of an anastomosis device 400, in the resting state, that is adapted to necrose tissue. In this embodiment, each petal 406 can be formed by adjacent arms 410, 412 connected by a tip 414 that extends along a portion of the outer periphery 408 of the device 400. As illustrated in FIGS. 4B and 4C, the adjacent arms 410, 412 of each petal 406 can be curved relative to a plane 427 of the device 200. For example, each of the arms 410', 412' of the superior set of petals 406' can include an arc portion 430 that extends away from the plane 427 in a first longitudinal direction 433 and a bend portion 434 that orients the tip 414' substantially parallel to the plane 427 of the device 400. Additionally, each of the arms 410", 412" of the inferior set of petals 406" can include an arc portion 434 that extends away from the plane 427 in a second longitudinal direction 435 and a bend portion 436 that orients the tip 414" substantially parallel to the plane 427 of the device 400. In use, the curved arc of the arms 410, 412 of the petals 406 can focus pressure applied by the petals 206 to the outer periphery 408 of the device 400.

The superior and inferior petals 406', 406" can also be positioned at particular locations relative to one another to facilitate necrosis formation at the outer periphery 408. As shown in FIG. 4A, opposed petals 406 are staggered relative to each other and portions of the tips 414 of adjacent petals 406 are configured to cross at multiple locations 426 about the circumference of the of the device 400. For example, each tip 414' of superior petal 406' crosses the tips 414" of two adjacent inferior petals 406" at locations 426-1, 26-2. Once deployed in tissue, opposing tips 414 can contact (e.g., clamp) tissue at each of the locations 426 and can apply a relatively large pressure (e.g., localized pressures calculated to be in the range of approximately 276 kPa (40 psi) to 483 kPa (70 psi)) to the tissue to limit or prevent blood flow thereto and to cause the tissue to become necrotic at the outer periphery 408 and inbound of the outer periphery 408. Also, the amount of overlap of the tips 414, relative to a circumference of the device 400, can affect the degree of tissue necrosis at the outer periphery 408. For example, in the embodiment of FIGS. 4A-4C, opposing petal tips 414 can overlap and clamp tissue along approximately 40% of the outer periphery 408 or circumference of the deployed device 400 and, as a result, can cause substantially uniform necrosis of the tissue about the outer periphery 408, thereby allowing the anastomosis device 400 as well as the necrotic tissue to separate from the healthy tissue and pass through the digestive system of a patient.

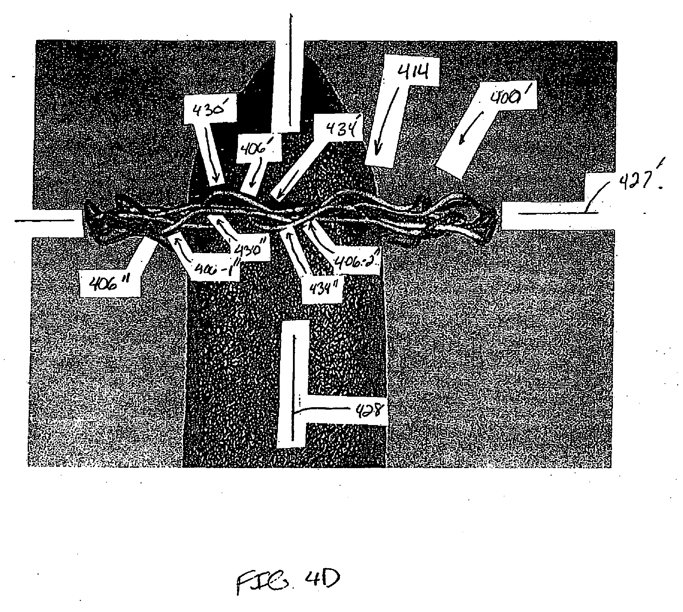

One skilled in the art will understand that the tips 414 of the petals 406 can be configured in a variety of different ways. In one embodiment of the device 400', as shown in FIG. 4D, the tips 414 of the petals 406', 406" can be arced or curved relative to the plane 427' of the device 400'. For example, the tip 414 of each of the superior and inferior petals 406', 406" has a peak portion 430', 430" that defines an inferior facing opening and a trough portion 434', 434" that defines a superior facing opening. With the opposed superior and inferior petals 406', 406" petals being staggered relative to each other, the peaks 430', 430" and troughs 434', 434" of opposed superior and inferior petals 406', 406" can be aligned along the outer periphery. For example, a peak portion 430' of the superior petal 406' can be aligned with a peak portion 430" of a first inferior petal 406-1" and a trough portion 434' of the superior petal 406' can be aligned with a trough portion 434" of a second inferior petal 406-2". The overlapping peaks 430', 430" and troughs 434', 434" of the opposed petals 406', 406" form a relatively long circumferential path about the outer periphery 408. As such, tissue disposed at the outer periphery 408 can become stretched by the overlapping peaks 430', 430" and troughs 434', 434" of the opposed petals 406', 406" and, as a result, the thickness of the affected tissue will decrease. Such a decrease in tissue thickness can result in the tissue being exposed to relatively high pressures as applied by the device 400. In use, the overlapping peaks 430', 430" and troughs 434', 434" of opposed petals 406', 406" can clamp tissue along approximately 50% of the outer periphery 408 ofthe device 400' and can apply a relatively large force (e.g., localized pressures calculated to be in the range of about 517 to 552 kPa (75 to 80 psi)) to tissue disposed at the outer periphery 408 to cause substantially uniform necrosis of the tissue.

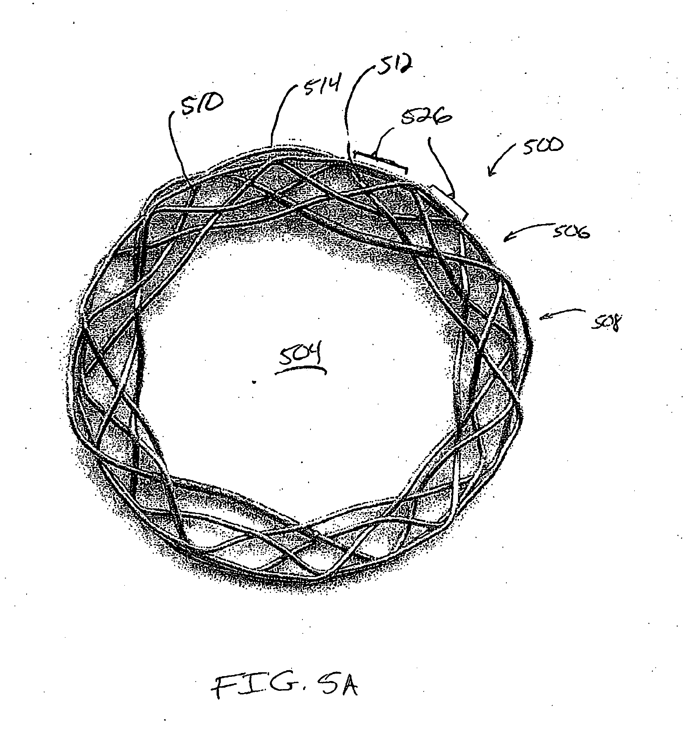

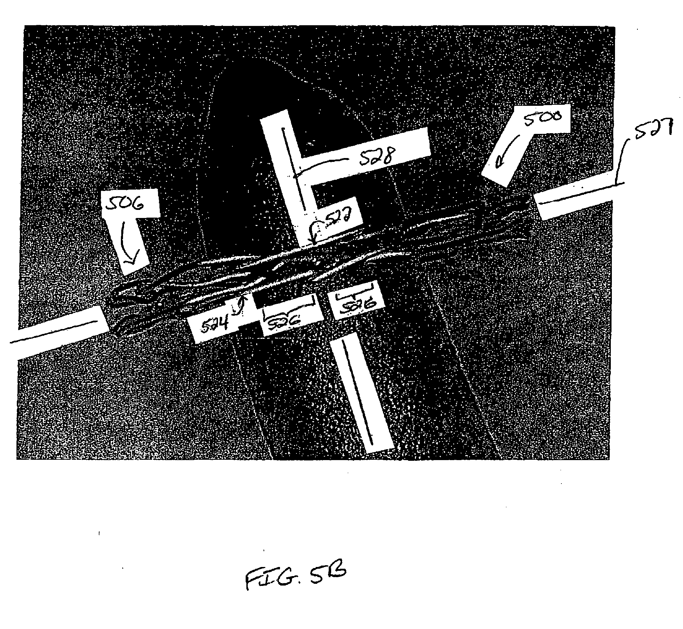

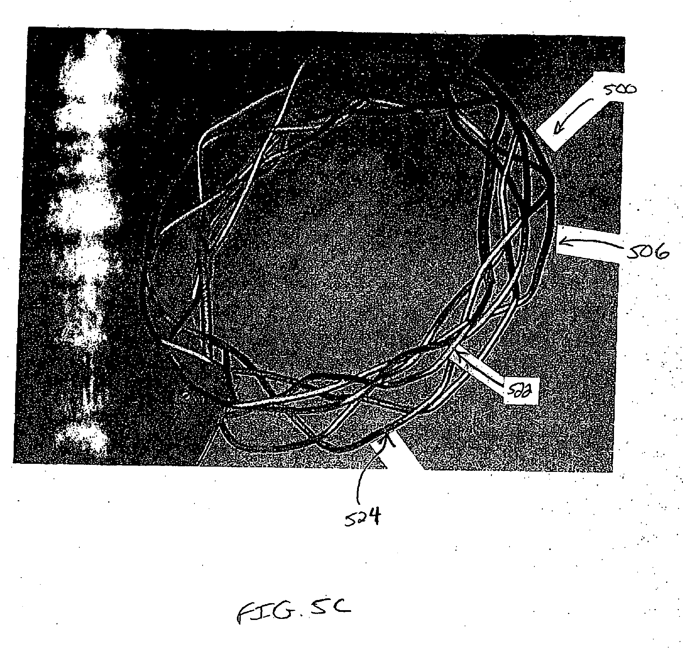

FIGS. 5A-5C illustrate an embodiment of an anastomosis device 500, in the resting state, having opposed petals 506 in which the adjacent arms 510, 512 and tip 514 of each petal 506 are oriented in a substantially planar configuration and where the petal tips 514 of the opposed petals 506 have midpoints 522, 524 that are staggered or offset relative to each other. As shown in FIG. 5B, the arms 510, 512 and tips 514 are oriented to be substantially parallel to a plane 527 that is perpendicular to a central axis 528 of the device 500. Further, the tips 514 of the opposing petals 506 cross at multiple locations 526 about an outer periphery 508 of the device 500. By way of example, the crossing portions 526 of the tips 514 can overlap and clamp tissue along approximately 85% of the outer periphery 508 of the deployed device 500.

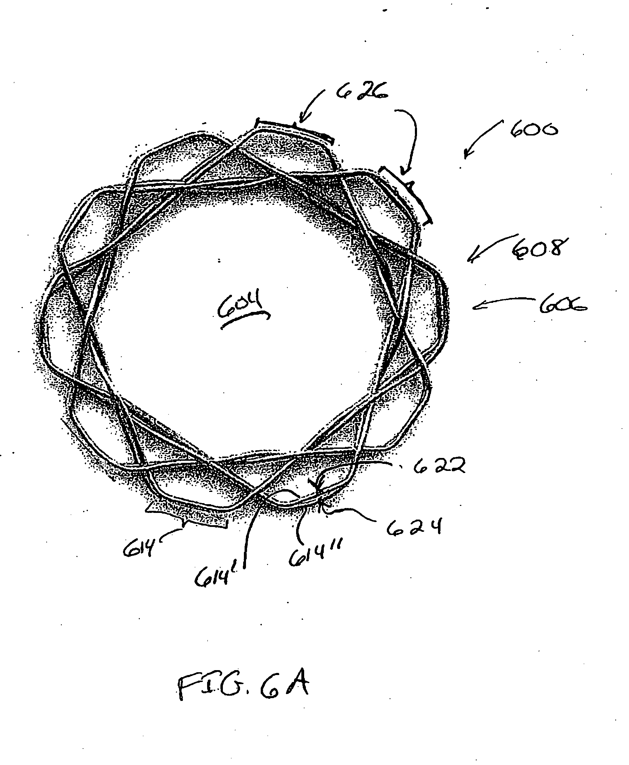

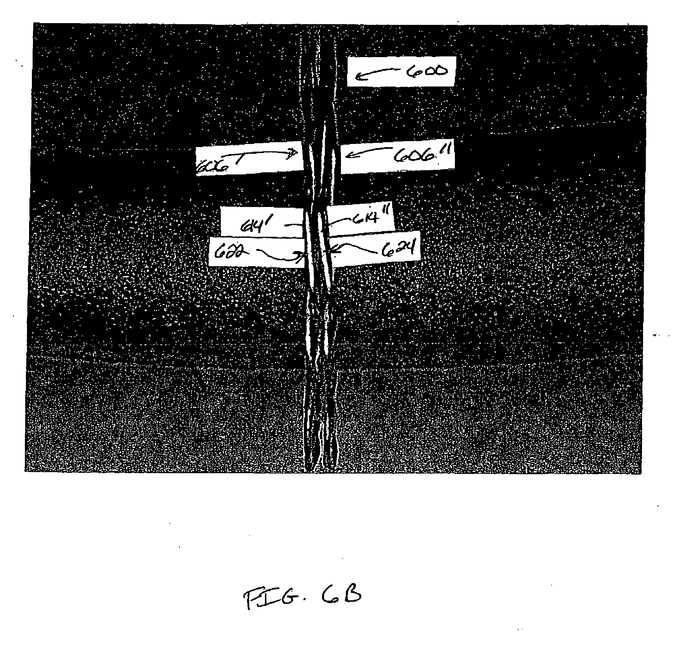

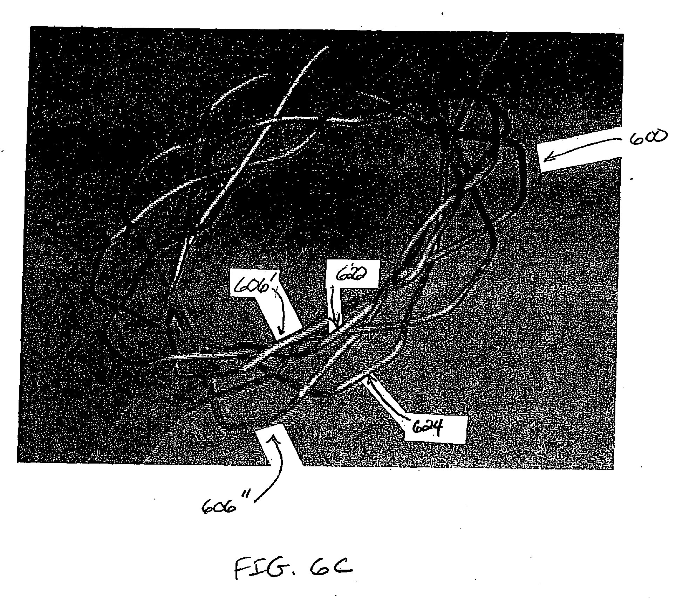

FIGS. 6A-6C illustrate another embodiment of an anastomosis device 600 that is adapted to necrose tissue. In this embodiment, opposed petals 606 are substantially aligned with each otherabout an outer periphery 608 of the device 600. That is, the tips 614' of each of the superior petals 606' have midpoints 622 that are substantially aligned with midpoints 624 of the tips 614" of the inferior petals 606". As a result, the tips 614 of the aligned superior and inferior petals 406', 406" cross at multiple locations 626 about the outer periphery 608 of the of the device 600 to clamp tissue along approximately 77% of the outer periphery 608. In use, opposing tips 614', 614" can clamp tissue at each of the locations 626 to apply a relatively large pressure (e.g., localized pressures calculated to be in the range of about 138 to 172 kPa (20 to 25 psi)) to the tissue to limit or prevent blood flow thereto and to cause the clamped tissue to become necrotic.

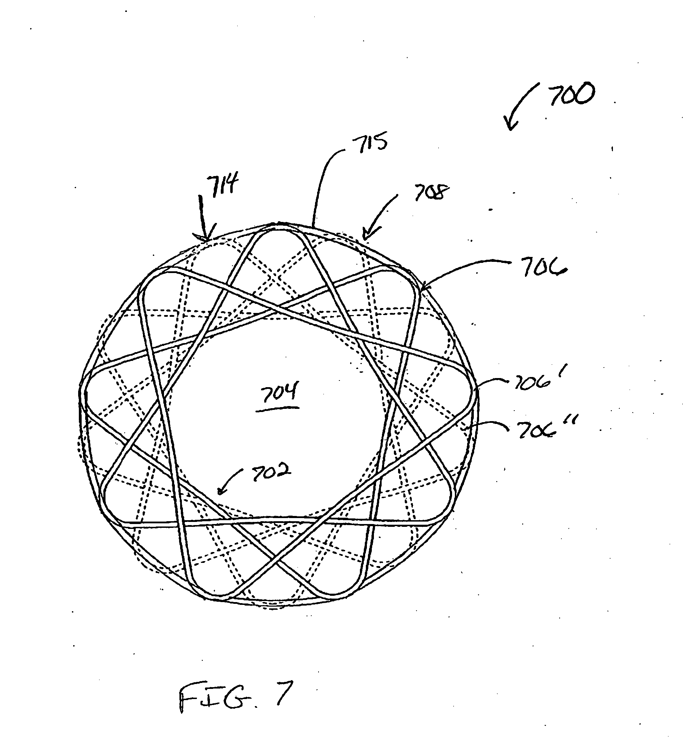

FIG. 7 illustrates another embodiment of an anastomosis device 700, in a resting state, that is adapted to necrose tissue. As shown, the anastomosis device 700 has an annular, ring-shaped configuration with an inner periphery 702 that defines an opening 704 and petals 706 extending from the inner periphery 702 and defining an outer periphery 708. The inner periphery 702 of the device 700 is defined by overlapping of the meshed wire and the outer periphery 708 is defined by the tips 714 of the superior and inferior petals 706. The outer periphery 708 of the anastomosis device 700 also includes opposing pressure distribution rings 715 disposed about to the tips 714 of each set of the opposed petals 716. For example, the device 700 includes an upper ring 715 disposed about the tips 714 of the superior petals 706' and a lower ring (not shown) disposed about the tips 714 of the inferior petals 706". In use, the pressure distribution rings 715 can distribute pressure from the petal tips 714 to tissue disposed substantially along the entire outer periphery 708 of the device 700 to create a substantially uniform blood flow barrier about the outer periphery 708 of the device 700. For example, the anastomosis device 700 can clamp tissue along approximately 90% of the outer periphery 708 to apply a substantially uniform pressure of approximately 241 kPa (35 psi) to the tissue disposed at the outer periphery 708.

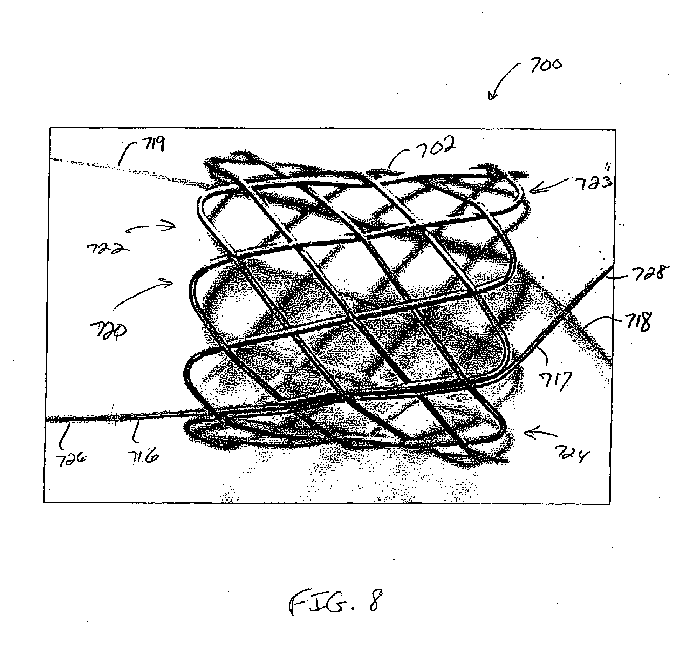

One skilled in the art will understand that the pressure distribution rings 715 can be formed at the outer periphery 708 of the device 700 in a number of ways. For example, as described below with respect to FIG. 8, when the anastomosis device 700 is manufactured, two wires can be woven together to form a mesh wall 702 such that, at the end of the weaving process, portions 716, 717, 718, 719 of the two wires can extend from the device 700. Two of the end wires can then be removed from the device 700 while the remaining two end wires can be used to form the pressure distribution rings 715. For example, the wire portions 718 an 719 can be removed from the mesh wall 702, the wire portion 716 can be interwoven with the tips 720 of a first end 722 ofthe device 700 to form a first pressure distribution ring, and the wire portion 719 can be interwoven with the tips 723 of a second end 724 of the device 700 to form a second pressure distribution ring. Once the wire portions 716, 719 have been woven to form the pressure distribution rings, the respective free ends 726, 728 ofthe wire portions 716, 719 can remain uncoupled to the mesh wall 702. In use, as the device 700 is deployed from an applier and collapses from an expanded, tubular shape to an annular, deployed shape, the free ends 726, 728 of the wire portions 716, 719 can slide through the tips 720, 723 to allow the pressure distribution rings 715 of the device 700 to expand from a collapsed state to an expanded state. When deployed, the pressure distribution rings 715 can distribute pressure from the petal tips 714 to tissue disposed substantially along the entire outer periphery 708 of the device 700.

While the pressure distribution rings 715 can be formed at the outer periphery 708 of the device 700 using two of the wire portions 716, 717, 718, 719 that extend from the device 700, one skilled in the art will understand that the pressure distribution rings 715 can be formed from separate wires that are added to the device 700. For example, at the end of the weaving process, all of the wire portions 716, 717, 718, 719 can be removed from the device 700 and separate wire elements can be attached. In particular, wire elements having a larger or smaller diameter than that of the wire forming the mesh wall 702 or wire elements formed of a material that is different than the wire forming the mesh wall 702 can be attached to the device 700.

The various anastomosis devices described above are adapted to cause necrosis of the clamped tissue. It is sometimes desirable, however, for an anastomosis device to allow tissue to overgrow the device at an anastomosis site. A non-necrosing anastomosis device is similar to necrosing anastomosis devices in that it has a ring shaped configuration in a resting or deployed state with an inner and outer periphery.

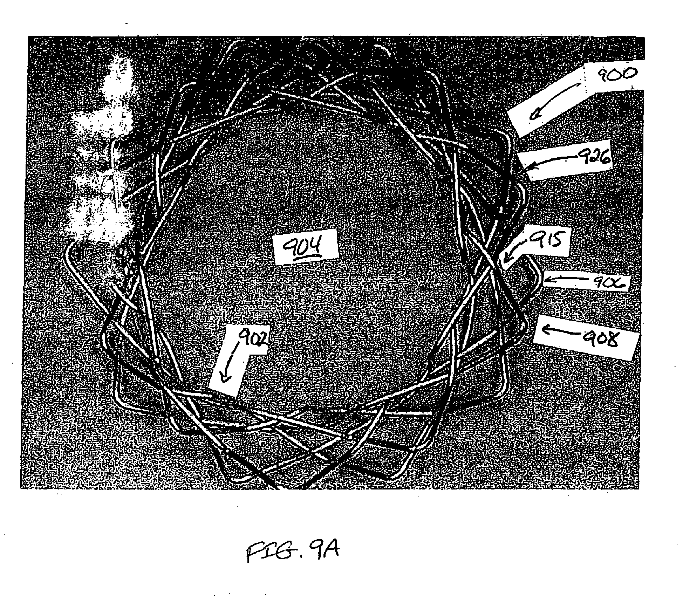

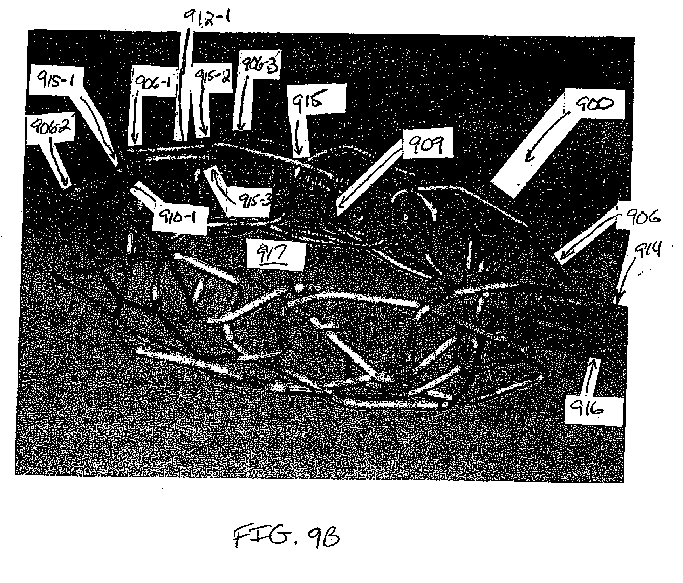

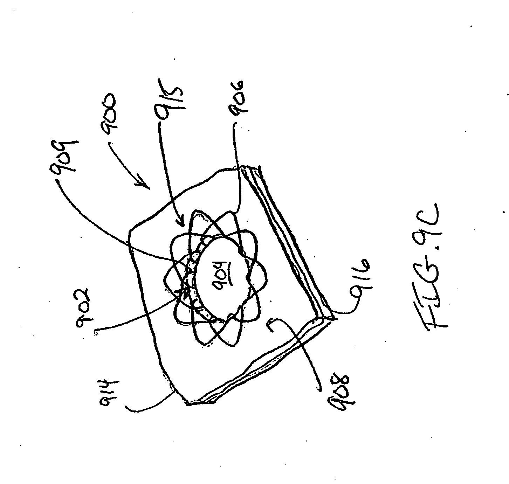

FIGS. 9A-9C show one embodiment of a non-necrosing type anastomosis device 900 in a resting or deployed state. As shown, the anastomosis device 900 has an annular, ring-shaped configuration with an inner periphery 902 that defines an opening 904 and petals 906 extending from the inner periphery 902 and defining an outer periphery 908. One characteristic of such a non-necrosing anastomosis device 900 is that the inner periphery 902 of the device 900 is formed from nonoverlapping wire segments 909. For example, as illustrated in FIG. 9B, the device 900 can be woven such that in the deployed state the wire segments 909 disposed at the inner periphery 902 of the device 900, within a lumen 917 formed between opposed tissues 914, 916, do not contact each other. Instead, the wires forming the arms of adjacent superior or inferior petals 906 can contact each other at locations 915. For example as illustrated in FIG. 9B, an arm 912-1 of a first superior petal 906-1 is disposed beneath an arm of a second superior petal 906-2 at location 915-1, an arm 910-1 of the first superior petal 906-1 is disposed over an arm of a third superior petal 906-3 at location 915-2, and the arms of the second and third superior petals 906-2, 906-3 contact each other at location 915-3. It is believed that such a design discourages the growth of bacteria or biofilms which can limit or prevent tissue growth due to the lack of contact between wire segments 909 at the inner periphery. Thus, tissue at the inner periphery 902 is able to grow over the wire segments 909. Accordingly, when the device 900 is in the deployed state as shown in FIG. 9C, the wire segments 909 forming the inner periphery 902 maintain a passageway between the two apposed tissue layers 914, 916 at an anastomosis site to allow fluid and/or other substances to be passed between the layers 914, 916 and promote tissue overgrowth of the segments 909 at the inner periphery 902. The device 900 still enables the superior and inferior petals 906 to engage the tissue layers 914, 916 and apply a pressure to the layers 914, 916 that is sufficient to maintain apposition of the tissue layers 914, 916 but that is below a threshold that can cause the tissues to become necrotic. For example, the opposing superior and inferior petals 906', 906" can overlap at locations 926 about the outer periphery 908 to apply a pressure of less than about 14 kPa (2 psi) to the tissue layers 914, 916 at each of the locations 926.

One skilled in the art will appreciate further features and advantages of the invention based on the above-described embodiments. Accordingly, the invention is not to be limited by what has been particularly shown and described, except as indicated by the appended claims.

## Claims

1. An anastomosis device (200), comprising:
a body formed from a woven shape-memory wire and defining a central lumen (204), the body being configurable between an expanded position in which the body assumes a generally tubular configuration having a wire mesh wall (242) adapted for insertion into a lumen of an anastomotic site and a rest position in which the body assumes an annular configuration having a plurality of petals (206) with petal tips (214) that define an outer periphery (208), the rest position being effective to hold opposed tissues of the anastomotic site in apposition and to apply a pressure to the opposed tissues such that the pressure decreases from the outer periphery (208) to an inner periphery (202) of the device,
wherein the plurality of petals (206) comprise a superior set (206') of adjacent petals and an inferior set (206") of adjacent petals, and the device is adapted to receive the opposed tissues between the inferior and superior sets of petals,
wherein, in a rest position, each petal of the superior set (206') and inferior set (206") of adjacent petals is formed by adjacent arms (210, 212) connected by a tip (214) that extends along a portion of the outer periphery (208) of the device, the tip (214) having a first radius (216) and each arm being connected to the tip at a bend (218) that has a second radius (220), the first radius being greater than the second radius,
**characterised in that** the adjacent arms (210, 212) of each petal (206) are oriented in a plane (227) that is substantially perpendicular to a central axis (228) extending through the central lumen (204) of the device, at least a portion of the adjacent arms (210', 212') of the superior set (206') of petals including an arc portion (230) having an inferior facing opening (232), and at least a portion of the adjacent arms (210", 212") of the inferior set (206") of petals including an arc portion (234) having a superior facing opening (236).

2. The anastomosis device of claim 1, wherein the adjacent arms (410', 412') of the superior set (406') of petals include a bend portion (434) disposed between the arc portion (430) and the tip (414'), the bend portion (434) of the adjacent arms orienting the tip in the plane (427) that is substantially perpendicular to a central axis (428) extending through the central lumen of the device and wherein the adjacent arms (410", 412") of the inferior set (406") of petals include a bend portion (436) disposed between the arc portion (432) and the tip (414"), the bend portion of the adjacent arms orienting the tip in the plane that is substantially perpendicular to a central axis extending through the central lumen of the device.

3. An anastomosis device (400, 400'), comprising:
a body formed from a woven shape-memory wire and defining a central lumen (204), the body being configurable between an expanded position in which the body assumes a generally tubular configuration having a wire mesh wall (242) adapted for insertion into a lumen of an anastomotic site and a rest position in which the body assumes an annular configuration having a plurality of petals (206) with petal tips (214) that define an outer periphery (208), the rest position being effective to hold opposed tissues of the anastomotic site in apposition and to apply a pressure to the opposed tissues such that the pressure decreases from the outer periphery (208) to an inner periphery (202) of the device,
wherein the plurality of petals (206) comprise a superior set (206') of adjacent petals and an inferior set (206") of adjacent petals, and the device is adapted to receive the opposed tissues between the inferior and superior sets of petals,
wherein, in a rest position, each petal of the superior set (206') and inferior set (206") of adjacent petals is formed by adjacent arms (210, 212) connected by a tip (214) that extends along a portion of the outer periphery (208) of the device, the tip (214) having a first radius (216) and each arm being connected to the tip at a bend (218) that has a second radius (220), the first radius being greater than the second radius, **characterised in that**
the tip (414) of each petal (406', 406") has a peak portion (430', 430") defining an inferior facing opening and a trough portion (434', 434") defining a superior facing opening.

4. The anastomosis device of claim 1 or 3, wherein the tips (214') of the superior set (206') of adjacent petals have midpoints (222) that are staggered about a circumference of the device relative to midpoints (224) of the tips (214") of the inferior set (206") of adjacent petals.

5. The anastomosis device of claim 1 or 3, wherein the tips (614') of the superior set (606') of adjacent petals have midpoints (622) that are substantially aligned with midpoints (624) of the tips (614") of the inferior set (606") of adjacent petals.

6. The anastomosis device of claim 1 or 3 wherein the tips (214) of the petals (206) are adapted to contact the opposed tissues along a distance greater than approximately 5% of the circumference of the outer periphery (208) of the device (200) when configured in the rest position.

## Patentansprüche

1. Anastomose-Vorrichtung (200), umfassend:
einen Körper, der aus einem gewebten Formgedächtnisdraht geformt ist und ein zentrales Lumen (204) definiert, wobei der Körper zwischen einer expandierten Position, in welcher der Körper eine allgemein röhrenförmige Konfiguration mit einer Drahtgeflechtwand (242), die zur Einführung in ein Lumen einer Anastomosenstelle ausgelegt ist, und einer Ruhestellung, in welcher der Körper eine ringförmige Konfiguration mit einer Vielzahl von Blättern (206) mit Blattspitzen (214), die einen Außenumfang (208) definieren, annimmt, konfigurierbar ist, wobei die Ruhestellung gegenüberliegende Gewebe der Anastomosenstelle effektiv in Apposition halten kann und einen Druck auf die gegenüberliegenden Gewebe aufbringen kann, so dass der Druck von dem Außenumfang (208) zu einem Innenumfang (202) der Vorrichtung abnimmt,
worin die Vielzahl von Blättern (206) einen oberen Satz (206') von benachbarten Blättern und einen unteren Satz (206") von benachbarten Blättern umfasst, und die Vorrichtung zur Aufnahme der gegenüberliegenden Gewebe zwischen den unteren und oberen Sätzen von Blättern ausgelegt ist,
worin jedes Blatt des oberen Satzes (206') und des unteren Satzes (206") von benachbarten Blättern in der Ruhestellung von benachbarten Armen (210, 212), die über eine Spitze (214), die sich entlang eines Abschnitts des Außenumfangs (208) der Vorrichtung erstreckt, verbunden sind, geformt wird, wobei die Spitze (214) einen ersten Radius (216) aufweist und jeder Arm mit der Spitze an einer Biegung (218) verbunden ist, die einen zweiten Radius (220) aufweist, wobei der erste Radius größer als der zweite Radius ist,
**dadurch gekennzeichnet, dass** die benachbarten Arme (210, 212) jedes Blatts (206) in einer Ebene (227) orientiert sind, die im Wesentlichen senkrecht zu einer Mittelachse (228) ist, die sich durch das zentrale Lumen (204) der Vorrichtung erstreckt, wobei zumindest ein Teil der benachbarten Arme (210', 212') des oberen Satzes (206') von Blättern einen Bogenabschnitt (230) mit einer nach unten weisenden Öffnung (232) aufweist und zumindest ein Teil der benachbarten Arme (210", 212") des unteren Satzes (206") von Blättern einen Bogenabschnitt (234) mit einer nach oben weisenden Öffnung (236) aufweist.

2. Anastomose-Vorrichtung nach Anspruch 1, worin die benachbarten Arme (410', 412') des oberen Satzes (406') von Blättern einen gebogenen Abschnitt (434) aufweisen, der zwischen dem Bogenabschnitt (430) und der Spitze (414') angeordnet ist, wobei der gebogene Abschnitt (434) der benachbarten Arme die Spitze in der Ebene (427) orientiert, die im Wesentlichen senkrecht zu einer Mittelachse (428) ist, die sich durch das zentrale Lumen der Vorrichtung erstreckt, und worin die benachbarten Arme (410", 412") des unteren Satzes (406") von Blättern einen gebogenen Abschnitt (436) aufweisen, der zwischen dem Bogenabschnitt (432) und der Spitze (414") angeordnet ist, wobei der gebogene Abschnitt der benachbarten Arme die Spitze in der Ebene orientiert, die im Wesentlichen senkrecht zu einer Mittelachse ist, die sich durch das zentrale Lumen der Vorrichtung erstreckt.

3. Anastomose-Vorrichtung (400, 400'), umfassend:
einen Körper, der aus einem gewebten Formgedächtnisdraht geformt ist und ein zentrales Lumen (204) definiert, wobei der Körper zwischen einer expandierten Position, in welcher der Körper eine allgemein röhrenförmige Konfiguration mit einer Drahtgeflechtwand (242), die zur Einführung in ein Lumen einer Anastomosenstelle ausgelegt ist, und einer Ruhestellung, in welcher der Körper eine ringförmige Konfiguration mit einer Vielzahl von Blättern (206) mit Blattspitzen (214), die einen Außenumfang (208) definieren, annimmt, konfigurierbar ist, wobei die Ruhestellung gegenüberliegende Gewebe der Anastomosenstelle effektiv in Apposition halten kann und einen Druck auf die gegenüberliegenden Gewebe aufbringen kann, so dass der Druck von dem Außenumfang (208) zu einem Innenumfang (202) der Vorrichtung abnimmt,
worin die Vielzahl von Blättern (206) einen oberen Satz (206') von benachbarten Blättern und einen unteren Satz (206") von benachbarten Blättern umfasst, und die Vorrichtung zur Aufnahme der gegenüberliegenden Gewebe zwischen den unteren und oberen Sätzen von Blättern ausgelegt ist,
worin jedes Blatt des oberen Satzes (206') und des unteren Satzes (206") von benachbarten Blättern in der Ruhestellung von benachbarten Armen (210, 212), die über eine Spitze (214), die sich entlang eines Abschnitts des Außenumfangs (208) der Vorrichtung erstreckt, verbunden sind, geformt wird, wobei die Spitze (214) einen ersten Radius (216) aufweist und jeder Arm mit der Spitze an einer Biegung (218) verbunden ist, die einen zweiten Radius (220) aufweist, wobei der erste Radius größer als der zweite Radius ist,
**dadurch gekennzeichnet, dass** die Spitze (414) jedes Blatts (406 `, 406") einen Gipfelabschnitt (430', 430"), der eine nach unten weisende Öffnung definiert, und einen Muldenabschnitt (434', 434") aufweist, der eine nach oben weisende Öffnung definiert.

4. Anastomose-Vorrichtung nach Anspruch 1 oder 3, worin die Spitzen (214') des oberen Satzes (206') von benachbarten Blättern Mittelpunkte (222) aufweisen, die um einen Umfang der Vorrichtung relativ zu Mittelpunkten (224) der Spitzen (214") des unteren Satzes (206") von benachbarten Blättern versetzt sind.

5. Anastomose-Vorrichtung nach Anspruch 1 oder 3, worin die Spitzen (614') des oberen Satzes (606') von benachbarten Blättern Mittelpunkte (622) aufweisen, die im Wesentlichen mit Mittelpunkten (624) der Spitzen (614") des unteren Satzes (606") von benachbarten Blättern ausgerichtet sind.

6. Anastomose-Vorrichtung nach Anspruch 1 oder 3, worin die Spitzen (214) der Blätter (206) ausgelegt sind, die gegenüberliegenden Gewebe entlang einer Strecke, die größer als ungefähr 5% des Umfangs des Außenumfangs (208) der Vorrichtung (200) in der Ruhestellung ist, zu berühren.

## Revendications

1. Dispositif (200) d'anastomose, comportant :
un corps formé dans un fil métallique tissé à mémoire de forme et délimitant une lumière (204) centrale, le corps pouvant être configuré entre une position étendue dans laquelle le corps prend une configuration généralement tubulaire présentant une paroi (242) en treillis métallique conçue pour être introduite dans une lumière d'un site anastomotique et une position de repos dans laquelle le corps prend une configuration annulaire présentant une pluralité de pétales (206) comportant des sommets (214) de pétales qui délimitent un pourtour (208) externe, la position de repos servant à maintenir les tissus opposés du site anastomotique en apposition et à appliquer une pression sur les tissus opposés de sorte que la pression diminue du pourtour (208) externe à un pourtour (202) interne du dispositif,
la pluralité de pétales (206) comportant un ensemble (206') supérieur de pétales adjacents et un ensemble (206") inférieur de pétales adjacents, et le dispositif étant conçu pour recevoir les tissus opposés entre les ensembles inférieur et supérieur de pétales,
dans une position de repos, chaque pétale de l'ensemble (206') supérieur et de l'ensemble (206") inférieur de pétales adjacents étant formé par des bras (210, 212) adjacents reliés par un sommet (214) qui s'étend le long d'une partie du pourtour (208) externe du dispositif, le sommet (214) ayant un premier rayon (216) et chaque bras étant relié au sommet au niveau d'une courbure (218) qui a un second rayon (220), le premier rayon étant supérieur au second rayon,
**caractérisé en ce que** les bras (210, 212) adjacents de chaque pétale (206) sont orientés dans un plan (227) qui est sensiblement perpendiculaire à un axe (228) central s'étendant dans la lumière (204) centrale du dispositif, au moins une partie des bras (210', 212') adjacents de l'ensemble (206') supérieur de pétales comprenant une partie (230) arc présentant une ouverture (232) qui est opposée à l'ensemble inférieur de pétales, et au moins une partie des bras (210", 212") adjacents de l'ensemble (206") inférieur de pétales comprenant une partie (234) arc présentant une ouverture (236) qui est opposée à l'ensemble supérieur de pétales.

2. Dispositif d'anastomose selon la revendication 1, les bras (410', 412') adjacents de l'ensemble (406') supérieur de pétales comprenant une partie (434) courbure disposée entre la partie (430) arc et le sommet (414'), la partie (434) courbure des bras adjacents orientant le sommet dans le plan (427) qui est sensiblement perpendiculaire à un axe (428) central s'étendant dans la lumière centrale du dispositif et les bras (410", 412") adjacents de l'ensemble (406") inférieur de pétales comprenant une partie (436) courbure disposée entre la partie (432) arc et le sommet (414"), la partie courbure des bras adjacents orientant le sommet dans le plan qui est sensiblement perpendiculaire à un axe central s'étendant dans la lumière centrale du dispositif.

3. Dispositif (400, 400') d'anastomose comportant :
un corps formé dans un fil métallique tissé à mémoire de forme et délimitant une lumière (204) centrale, le corps pouvant être configuré entre une position étendue dans laquelle le corps prend une configuration généralement tubulaire présentant une paroi (242) en treillis métallique conçue pour être introduite dans une lumière d'un site anastomotique et une position de repos dans laquelle le corps prend une configuration annulaire présentant une pluralité de pétales (206) comportant des sommets (214) de pétales qui délimitent un pourtour (208) externe, la position de repos servant à maintenir les tissus opposés du site anastomotique en apposition et à appliquer une pression sur les tissus opposés de sorte que la pression diminue du pourtour (208) externe à un pourtour (202) interne du dispositif,
la pluralité de pétales (206) comporte un ensemble (206') supérieur de pétales adjacents et un ensemble (206") inférieur de pétales adjacents, et le dispositif est conçu pour recevoir les tissus opposés entre les ensembles inférieur et supérieur de pétales,
dans une position de repos, chaque pétale de l'ensemble (206') supérieur et de l'ensemble (206") inférieur de pétales adjacents étant formé par des bras (210, 212) adjacents reliés par un sommet (214) qui s'étend le long d'une partie du pourtour (208) externe du dispositif, le sommet (214) ayant un premier rayon (216) et chaque bras étant relié au sommet au niveau d'une courbure (218) qui a un second rayon (220), le premier rayon étant supérieur au second rayon, **caractérisé en ce que**
le sommet (414) de chaque pétale (406', 406") présente une partie (430', 430") crête délimitant une ouverture qui est opposée à l'ensemble inférieur de pétales et une partie (434', 434") creux délimitant une ouverture qui est opposée à l'ensemble supérieur de pétales.

4. Dispositif d'anastomose selon la revendication 1 ou 3, les sommets (214') de l'ensemble (206') supérieur de pétales adjacents ont des points médians (222) qui sont décalés autour d'une circonférence du dispositif par rapport aux points médians (224) des sommets (214") de l'ensemble (206") inférieur de pétales adjacents.

5. Dispositif d'anastomose selon la revendication 1 ou 3, les sommets (614') de l'ensemble (606') supérieur de pétales adjacents ayant des points médians (622) qui sont sensiblement alignés avec les points médians (624) des sommets (614") de l'ensemble (606") inférieur de pétales adjacents.

6. Dispositif d'anastomose selon la revendication 1 ou 3, les sommets (214) des pétales (206) étant conçus pour venir en contact avec les tissus opposés sur une distance supérieure à environ 5 % de la circonférence du pourtour (208) externe du dispositif (200) lorsque configuré dans la position de repos.
